# EUROPEAN PATENT SPECIFICATION

(11) **EP 1 475 000 B1**
(45) Date of publication and mention of the grant of the patent: **25.03.2009**
(21) Application number: 04076370.8
(22) Date of filing: 06.05.2004
(51) Int. Cl.: A23L 3/16, A61L 2/00, A61L 2/04, A23C 3/03, C12H 1/00

(54) **A simplified multipurpose pasteurizer**
Einfacher Allzweckpasteurisator
Pasteurisateur simplifié à usage multiple

(30) Priority: 09.05.2003 IT mi20030936
(43) Date of publication of application: 10.11.2004
(73) Proprietor: Telme S.P.A., 26845 Codogno (IT)
(72) Inventor: Cigolini, Aldo, 26861 Retegno - Fombio (Lodi) (IT)
(74) Representative: De Gregori, Antonella

(56) References cited:
- EP-A- 0 230 194
- EP-A- 0 770 333
- EP-A- 0 832 570
- EP-A- 1 421 845
- WO-A-99/54227
- FR-A- 2 552 634
- GB-A- 394 455
- RICHARDSON P S ET AL: "HEAT PROCESSING EQUIPMENT" PROCESSING AND PACKAGING OF HEAT PRESERVED FOODS, XX, XX, 1991, pages 50-69, XP000541916

## Description

The present invention relates to a simplified multipurpose pasteurizer.

At the current state of the art, in pasteurizers treatment is envisaged of a certain pre-set number of litres of product, which entails an installation of a certain importance. Albeit in the presence of smaller amounts of product, there is however necessary a long time for obtaining the product required at the treatment temperature. For example, it is common to treat the product for approximately one and a half hours in order to reach the optimal temperature.

For the above purpose, in an insulating container, provided with a removable lid, there is set a tank, made of stainless steel, surrounded by a cooling coil. In addition, further coils must be arranged, which will enable circulation of a heating fluid to intervene for heating, whenever necessary, the product contained in the stainless-steel tank.

In this way, there are in any case involved intervention times such as the ones indicated above, which do not enable small amounts of product to be obtained in short times as required.

Furthermore, arrangement of the coils of the heating circuit and of the coils of the cooling circuit all around the tank that contains the product causes a wide dispersion both of the heat and of the cold with a useless consumption of energy.

Treatment of small amounts of products is consequently altogether economically disadvantageous both on account of the amounts of energy involved and on account of the times necessary for said treatment.

This type of problem is present both in round pasteurizers and in rectangular pasteurizers in the presence of mixers of various shapes and size.

Furthermore, it must not be forgotten that in some types of pasteurizers there is the need to treat ice-creams, which have components, such as fruit, that are delicate and involve the treatment of small amounts and require treatments of different types. Hence, once again there is the need to obtain a product in as short a time as possible and in extremely limited amounts.

FR 2 552 634 discloses a mobile pasteurising installation specially designed for canned foods.

The purpose of the present invention is hence to provide a pasteurizer that will enable, in the first place, all the drawbacks set forth above to be in general solved.

A further purpose of the present invention is to provide a pasteurizer that will enable wide versatility of use so that it is able to adapt both to strong demands and to demands of operation with a minimum energy involved, in the presence of extremely variable amounts of product to be treated.

The aforesaid purposes are achieved according to the present invention by a simplified multipurpose pasteurizer according to Claim 1.

The further claims define the additional characteristics of the invention.

The characteristics and advantages of a simplified multipurpose pasteurizer according to the present invention will emerge more clearly evident from the ensuing description, provided purely by non-limiting example, with reference to the attached schematic drawings, in which:
- Figure 1 is a schematic representation of a simplified multipurpose pasteurizer according to the present invention in a heating stage;
- Figure 2 is a schematic representation altogether similar to that of Figure 1 in a cooling stage; and
- Figures 3 and 4 are also schematic representations of a second embodiment of the pasteurizer according to the invention.

With reference in general to the figures, in which the same reference numbers designate elements that are the same as one another, there are illustrated alternative embodiments of a simplified multipurpose pasteurizer according to the present invention, which is set outside a treatment tank 11 and in which the ingredients that will form the mixture are introduced.

The tank 11, for example with round or in any case rectangular shape, equipped with a mixing pump (not illustrated) envisages a first opening 12 on its bottom for outlet of the mixture or product into a pipe 13. The pipe 13 passes through an exchanger 14, before reentering through a second opening 15 in the proximity of the bottom of the tank 11.

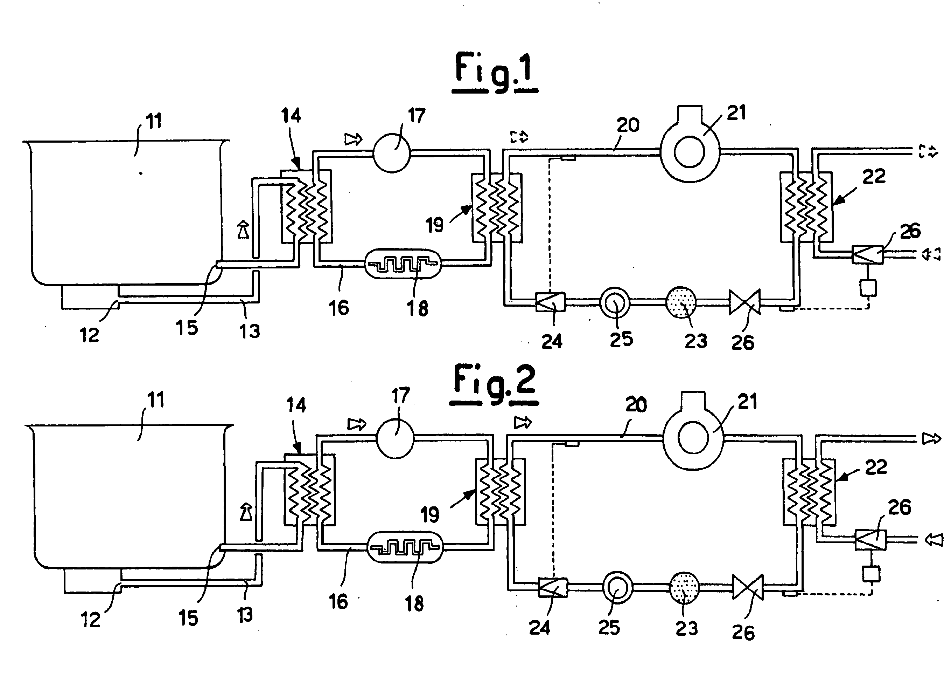

The exchanger 14, as illustrated in Figures 1 and 2, also receives a pipe 16 of a first closed circuit for circulation of glycol. This first circuit, which performs its primary operating step during the heating stage, envisages a motor-driven circulation pump 17 and a heating resistor 18. In addition, in a further exchange portion thereof, which is inactive during the heating stage, it is connected to an evaporator 19, connected, in turn, to a second closed circuit for the circulation of gas.

Said second circuit, which performs its primary operating step during the cooling stage and is set in series to the first circuit, also envisages a pipe 20, a compressor 21, a condenser 22, as well as a filter 23, and a thermostatic valve 24. In addition, there may be envisaged also accessory elements, such as warning lamps 25, further valves 26, etc.

It is evident that the first circuit in the cooling stage functions as auxiliary and connection element. In fact, it acts as area of heat exchange and enables cooling of the product that circulates in the pipe 13 thanks to the presence of the evaporator 19 and of the exchanger 14, in which the glycol that acts as cooling element passes.

The above particular pasteurizer according to the present invention is designed to extract the product to be treated from the tank and hence acts selectively on the amount being treated in a fast way and with considerable energy saving.

In fact, all the energy employed both in the heating stage and in the cooling stage acts directly on the product that is made to pass through the exchanger 14. In this way, the pasteurizer is exploited to the full, which, in really minimized times, is able to treat any amount of product.

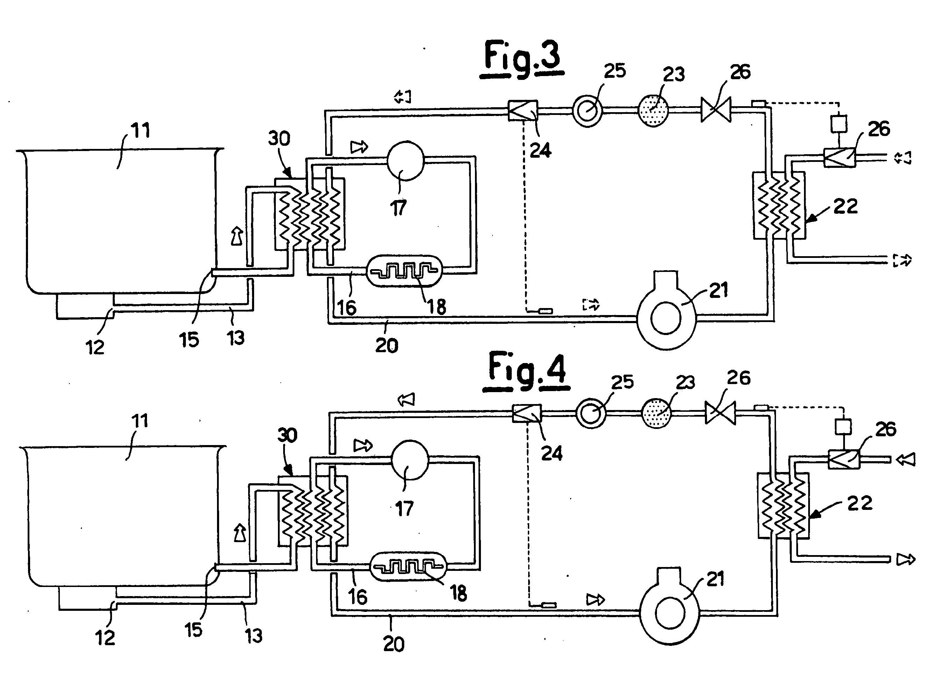

Figures 3 and 4 illustrate a second embodiment of a pasteurizer according to the present invention.

In said proposed alternative, in which, as has been said, elements that are the same as one another are designated by the same reference numbers, there is envisaged the location of an exchanger/evaporator element, designated by 30.

In the above exchanger/evaporator element 30, both the pipe 13 connected to the tank 11 and the pipes 16 and 20 connected, respectively, to the first and second circuits described above are made to pass.

Figures 3 and 4 show how the two circuits in this case are used alternatively and selectively thanks to the particular arrangement in parallel, and not in series as in the first embodiment.

Consequently, according to the present invention, it is possible to obtain a product or mixture in the times desired, and in times that are in any case decidedly limited, and in the desired amounts, thanks to the versatility of the pasteurizer thus conceived.

The pasteurizer hence presents the possibility of a considerable energy saving, at the same time presenting a high level of functionality.

The decidedly lower costs of the various components (motor-driven compressor, etc.) bring about also a considerable saving on the machine and on the investment costs.

## Claims

1. A simplified multipurpose method for pasteurizing a mixture or product contained in at least one tank (11) equipped with a mixing pump **characterized in that** it comprises the followings steps:
- extracting said mixture or product to be treated from said at least one tank (11) and introducing said mixture or product to be treated in a pipe (13), said at least one tank (11) having a first opening (12) on its bottom associated to said pipe (13),
- treating said mixture or product circulating in said pipe (13) outside said at least one tank (11), said pipe (13) outside the tank (11) being connected to a first circuit for heating (16, 17, 18) said mixture or product circulating in said pipe (13) and to a second circuit for cooling (20, 21, 22, 23, 24) of said mixture or product circulating in said pipe (13) via an exchanger (14, 30) and an evaporator (19, 30);
- reintroducing said mixture or product treated from said a pipe (13) to said at least one tank (11), said a pipe (13) being connected, in turn, to a second opening (15) said at least one tank (11).

2. A simplified multipurpose pasteurizer for pasteurizing a mixture or product contained in at least one tank (11)' according to method claim 1, **characterized in that** said pasteurizer comprises at least one tank (11) containing a mixture or product to be treated and respective heating and cooling circuits, said at least one tank (11) being equipped with a mixing pump and has a first opening (12) for the outlet of the mixture or product to be treated to a pipe (13) which is connected, in turn, to a second opening (15) for reentry of said mixture or product treated into said tank (11), said pipe (13) set outside the tank (11) being connected to a first circuit for heating (16, 17, 18) said mixture or product circulating in said pipe (13) and to a second circuit for cooling (20, 21, 22, 23, 24) of said mixture or product circulating in said pipe (13) via an exchanger (14, 30) and an evaporator (19, 30).

3. The pasteurizer according to claim 2, **characterized in that** said first and second circuits are set in series with respect to one another.

4. The pasteurizer according to claim 2, **characterized in that** said first and second circuits are set in parallel with respect to one another.

5. The pasteurizer according to claim 2, **characterized in that** said first circuit for heating is a glycol circuit, in which' there is provided a motor-driven circulation pump (17) and a heating resistor (18), as well as said exchanger (14), in which said pipe (13) connected at inlet to and outlet from said tank or drum (11) passes.

6. The pasteurizer according to claim 2, **characterized in that** said second circuit for cooling is a circuit for circulation of gas, which envisages a compressor (21), a condenser (22), a filter (23), and a thermostatic valve (24), as well as an evaporator (30), in which said pipe (13) connected at inlet to and outlet from said tank or drum (11) passes.

7. The pasteurizer according to claim 5, **characterized in that** said second circuit for cooling is a circuit for circulation of gas, which envisages a compressor (21), a condenser (22), a filter (23), and a thermostatic valve (24), as well as an evaporator (19), in which said pipe (20) of said first circuit passes.

## Patentansprüche

1. Vereinfachtes Mehrzweckverfahren zum Pasteurisieren einer Mischung oder eines Produktes, die/das in zumindest einem Tank (11) enthalten ist, der mit einer Mischpumpe ausgestattet ist, **dadurch gekennzeichnet, dass** es die folgenden Schritte umfasst:
- Entnehmen der zu behandelnden Mischung oder des zu behandelnden Produkts von dem zumindest einen Tank (11) und Einführen der zu behandelnden Mischung oder des zu behandelnden Produkts in ein Rohr (13), wobei der zumindest eine Tank (11) eine erste Öffnung (12) an seinem Boden besitzt, die dem Rohr (13) zugeordnet ist,
- Behandeln der Mischung oder des Produktes, die/das in dem Rohr (13) außerhalb des zumindest einen Tanks (11) zirkuliert, wobei das Rohr (13) außerhalb des Tanks (11) mit einem ersten Kreislauf zum Erwärmen (16, 17, 18) der Mischung oder des Produktes, die/das in dem Rohr (13) zirkuliert, und mit einem zweiten Kreislauf zum Kühlen (20, 21, 22, 23, 24) der Mischung oder des Produkts, die/das in dem Rohr (13) zirkuliert, über einen Tauscher (14, 30) und einen Verdampfer (19, 30) verbunden ist;
- Wiedereinführen der behandelten Mischung oder des behandelten Produktes von dem Rohr (13) zu dem zumindest einen Tank (11), wobei das Rohr (13) seinerseits mit einer zweiten Öffnung (15) des zumindest einen Tanks (11) verbunden ist.

2. Vereinfachter Mehrzweckpasteurisator zum Pasteurisieren einer Mischung oder eines Produkts, die/das in zumindest einem Tank (11) enthalten ist, gemäß Verfahrensanspruch 1, **dadurch gekennzeichnet, dass** der Pasteurisator zumindest einen Tank (11), der eine zu behandelnde Mischung oder ein zu behandelndes Produkt enthält, und jeweilige Heiz- und Kühlkreisläufe umfasst, wobei der zumindest eine Tank (11) mit einer Mischpumpe ausgestattet ist und eine erste Öffnung (12) für den Auslass der zu behandelnden Mischung oder des zu behandelnden Produkts zu einem Rohr (13) besitzt, das seinerseits mit einer zweiten Öffnung (15) zum Wiedereintritt der behandelten Mischung oder des behandelten Produktes in den Tank (11) verbunden ist, wobei das Rohr (13), das außerhalb des Tanks (11) eingerichtet ist, mit einem ersten Kreislauf zum Erwärmen (16, 17, 18) der Mischung oder des Produkts, die/das in dem Rohr (13) zirkuliert, und mit einem zweiten Kreislauf zum Kühlen (20, 21, 22, 23, 24) der Mischung oder des Produkts, die/das in dem Rohr (13) zirkuliert, über einen Tauscher (14, 30) und einen Verdampfer (19, 30) verbunden ist.

3. Pasteurisator nach Anspruch 2, **dadurch gekennzeichnet, dass** der erste und zweite Kreislauf in Reihe in Bezug zueinander eingerichtet sind.

4. Pasteurisator nach Anspruch 2, **dadurch gekennzeichnet, dass** der erste und zweite Kreislauf parallel in Bezug zueinander eingerichtet sind.

5. Pasteurisator nach Anspruch 2, **dadurch gekennzeichnet, dass** der erste Kreislauf zum Erwärmen ein Glykolkreislauf ist, in welchem eine motorbetriebene Zirkulationspumpe (17) und ein Heizwiderstand (18) wie auch der Tauscher (14) vorgesehen sind, in dem das Rohr (13), das an einem Einlass zu und einem Auslass von dem Tank oder einer Trommel (11) verbunden ist, verläuft.

6. Pasteurisator nach Anspruch 2, **dadurch gekennzeichnet, dass** der zweite Kreislauf zum Kühlen ein Kreislauf zur Zirkulation von Gas ist, in welchem ein Kompressor (21), ein Kondensator (22), ein Filter (23) und ein Thermostatventil (24) wie auch ein Verdampfer (30) vorgesehen sind, in dem das Rohr (13), das an einem Einlass zu und einem Auslass von dem Tank oder einer Trommel (11) verbunden ist, verläuft.

7. Pasteurisator nach Anspruch 5, **dadurch gekennzeichnet, dass** der zweite Kreislauf zum Kühlen ein Kreislauf zur Zirkulation von Gas ist, in welchem ein Kompressor (21), ein Kondensator (22), ein Filter (23) und ein Thermostatventil (24) wie auch ein Verdampfer (19) vorgesehen sind, in dem das Rohr (20) des ersten Kreislaufs verläuft.

## Revendications

1. Procédé polyvalent simplifié pour pasteuriser un mélange ou produit contenu dans au moins un réservoir (11) muni d'une pompe de mélange, **caractérisé en ce qu'**il comprend les étapes suivantes :
- extraction dudit mélange ou produit à traiter dudit au moins un réservoir (11) et introduction dudit mélange ou produit à traiter dans un tuyau (13), ledit au moins un réservoir (11) comportant une première ouverture (12) dans son fond associé audit tuyau (13),
- traitement dudit mélange ou produit circulant dans ledit tuyau (13) à l'extérieur dudit au moins un réservoir (11), ledit tuyau (13) à l'extérieur du réservoir (11) étant connecté à un premier circuit pour chauffer (16, 17, 18) ledit mélange ou produit circulant dans ledit tuyau (13) et à un deuxième circuit pour refroidir (20, 21, 22, 23, 24) ledit mélange ou produit circulant dans ledit tuyau (13) par l'intermédiaire d'un échangeur (14, 30) et d'un évaporateur (19, 30) ;
- réintroduction dudit mélange ou produit traité dudit tuyau (13) dans ledit au moins un réservoir (11), ledit tuyau (13) étant connecté, à son tour, à une deuxième ouverture (15) prévue dans ledit au moins un réservoir (11).

2. Pasteurisateur polyvalent simplifié pour pasteuriser un mélange ou produit contenu dans au moins un réservoir (11) selon la revendication 1, **caractérisé en ce que** ledit pasteurisateur comprend au moins un réservoir (11) contenant un mélange ou produit à traiter et des circuits de chauffage et de refroidissement respectifs, ledit au moins un réservoir (11) étant muni d'une pompe de mélange et comportant une première ouverture (12) pour la sortie du mélange ou produit à traiter vers un tuyau (13) qui est lui-même connecté à une deuxième ouverture (15) pour la réintroduction dudit mélange ou produit traité dans ledit réservoir (11), ledit tuyau (13) placé à l'extérieur du réservoir (11) étant connecté à un premier circuit pour chauffer (16, 17, 18) ledit mélange ou produit circulant dans ledit tuyau (13) et à un deuxième circuit pour refroidir (20, 21, 22, 23, 24) ledit mélange ou produit circulant dans ledit tuyau (13) par l'intermédiaire d'un échangeur (14, 30) et d'un évaporateur (19, 30).

3. Pasteurisateur selon la revendication 2, **caractérisé en ce que** lesdits premier et deuxième circuits sont montés en série l'un par rapport à l'autre.

4. Pasteurisateur selon la revendication 2, **caractérisé en ce que** lesdits premier et deuxième circuits sont montés en parallèle l'un par rapport à l'autre.

5. Pasteurisateur selon la revendication 2, **caractérisé en ce que** ledit premier circuit pour chauffer est un circuit de glycol, dans lequel il est prévu une pompe de circulation motorisée (17) et une résistance chauffante (18), ainsi que ledit échangeur (14), dans lequel passe ledit tuyau (13) connecté en entrée et en sortie dudit réservoir ou tambour (11).

6. Pasteurisateur selon la revendication 2, **caractérisé en ce que** ledit deuxième circuit pour refroidir est un circuit pour la circulation de gaz, qui renferme un compresseur (21), un condenseur (22), un filtre (23) et une vanne thermostatique (24), ainsi qu'un évaporateur (30), dans lequel passe ledit tuyau (13) connecté en entrée et en sortie dudit réservoir ou tambour (11).

7. Pasteurisateur selon la revendication 5, **caractérisé en ce que** ledit deuxième circuit pour refroidir est un circuit pour la circulation de gaz, qui renferme un compresseur (21), un condenseur (22), un filtre (23) et une vanne thermostatique (24), ainsi qu'un évaporateur (19), dans lequel passe ledit tuyau (20) dudit premier circuit.
